# EUROPEAN PATENT APPLICATION

(11) **EP 3 777 859 A1**
(43) Date of publication of application: **17.02.2021**
(21) Application number: 19776895.5
(22) Date of filing: 26.03.2019
(51) Int. Cl.: A61K 31/4545, A61P 7/06, A61P 13/12, A61P 27/02, A61P 43/00

(54) **THERAPEUTIC AGENT FOR LCAT DEFICIENCY**

(30) Priority: 30.03.2018 JP 2018068145
(71) Applicant: Daiichi Sankyo Company, Limited, Tokyo 103-8426 (JP)
(72) Inventor: SUGIDACHI Atsuhiro, Tokyo 103-8426 (JP); YAMADA Keisuke, Tokyo 103-8426 (JP)
(74) Representative: Fairbairn, Angus Chisholm
(86) International application number: PCT/JP2019/012637
(87) International publication number: WO 2019/189046

(57) **Abstract**

The present invention has an object of providing a pharmaceutical drug containing a compound having a good LCAT activation effect on a mutant LCAT protein and a therapeutic effect for LCAT deficiency, and also provides a therapeutic drug for LCAT deficiency containing a compound represented by Formula (I) 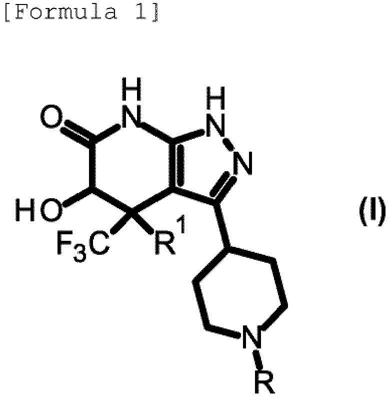 wherein R¹ is a hydrogen atom or a hydroxyl group, and R is a 2-(trifluoromethyl)pyrimidin-5-yl group or a 5-(trifluoromethyl)pyrazin-2-yl group, or a pharmacologically acceptable salt thereof.

## Description

### Technical Field

The present invention relates to a therapeutic drug for LCAT deficiency containing a pyrazolopyridine derivative having a good lecithin cholesterol acetyltransferase (hereinafter referred to as LCAT) activation effect (preferably a reversible LCAT activation effect) or a pharmacologically acceptable salt thereof.

### Background Art

Lecithin cholesterol acyl transferase (LCAT) deficiency is a genetic disease caused by genetic mutation of the enzyme LCAT responsible for cholesterol esterification, whereby free cholesterol and lecithin (phosphatidylcholine) are increased due to defective protein expression and/or decreased enzyme activity, resulting in the observation of a notable decrease in high density lipoprotein (HDL) cholesterol and a decrease in the serum cholesterol ester ratio. Abnormal lipoproteins with altered compositions deposited in tissues develop symptoms such as corneal opacity, renal disorder, and hemolytic anemia caused by an abnormal lipid composition of the erythrocyte membrane.

Pathological conditions caused by congenital dysfunction of LCAT are roughly categorized into familial LCAT deficiency (FLD) and fish eye disease (FED). Approximately 100 types of LCAT genetic mutations have been reported to date. Approximately 60 types are considered causative genes of FLD, of which 1/3 fail to produce full-length LCAT protein due to nonsense mutations or frameshift mutations, and 2/3 have activity with a partial deficiency due to missense mutations.

Reported as amino acid mutations associated with FLD are, for example, G30S, L32P, G33R, A93T, R135W, R135Q, R140H, R147W, Y156N, G183S, L209P, N228K, R244G, M252K, T321M, G344S, T347M, R399C (Non Patent Literature 1), G230R (Non Patent Literature 2), F382V, T208S (Non Patent Literature 3), S181N (Non Patent Literature 4), R140C (Non Patent Literature 5), G179R (Non Patent Literature 6), M293R (Non Patent Literature 7), P406L (Non Patent Literature 8), C74T (Non Patent Literature 9), R268L (Non Patent Literature 10), and D101N (Non Patent Literature 11).

Reported as amino acid mutations associated with FED are N131D, N391S, P10L, P10Q, T123I (Non Patent Literature 1), R99C (Non Patent Literature 12), T13M (Non Patent Literature 13), and the like.

FLD includes classic deficiency with a LCAT activity less than 10% of that of a healthy person and partial deficiency. In the classic deficiency, a large majority of cases result in the appearance of proteinuria mainly with albumin since early childhood and the development of progressive renal failure in the forties to fifties. The partial deficiency shows intermediate pathological conditions between the healthy condition and the classic deficiency, thereby showing a variety of clinical symptoms (degree of severity). There is a subtype of LCAT deficiency called FED developing only corneal opacity since comparatively early childhood. Vision impairment caused by the corneal opacity may require corneal transplantation. Both renal failure and corneal opacity cause major issues in terms of decreasing quality of life (QOL).

A factor determining life prognosis of LCAT deficiency is progress of renal failure. Renal transplantation has a temporary ameliorating effect on renal function but has a risk of recurrence. Diet therapy by low-fat meals for the purpose of protecting renal function and drug therapies by such as with angiotensin II receptor blockers and angiotensin-converting-enzyme inhibitors have been practiced, but all are symptomatic treatments and cannot meet expectations of ameliorating abnormal lipoprotein, which is the causative substance of renal function disorder, and their therapeutic and preventive effects on long-term extension of the pathological conditions are uncertain. At the moment, there is no established treatment method available (Non Patent Literature 14).

LCAT deficiency is caused by decreased LCAT activity and thus a method is demanded for continuously maintaining LCAT activity as a radical treatment. Studies conducted include enzyme replacement therapy by a recombinant LCAT protein (Non Patent Literature 15), gene therapy using an adeno-associated virus as a vector (Non Patent Literature 16) and ex vivo genetically engineered cell therapy by transplantation of LCAT gene transferred adipocytes (Non Patent Literature 17).

Recombinant protein replacement therapy necessitates practice of life-time periodical injections and poses issues typical of protein preparations such as the appearance of antibodies to the recombinant protein. Additionally, despite gene therapy and cell therapy using gene transferred cells having benefits of enabling continuous expression of LCAT protein and thus activity maintenance, decreased transgene expression in association with turnover of the gene transferred cells is anticipated, thereby leaving an issue in terms of stably retaining long-term LCAT activity.

An activator of LCAT proteins is reported as an approach different from LCAT protein replacement. As drugs that act on LCAT proteins and enhance the activity thereof are known peptide compounds (for example, Non Patent Literature 18) or low molecular compounds (for example, Patent Literature 1).

There is a report that the low molecular compound described in Patent Literature 1 was investigated using blood samples of healthy persons and patients with LCAT deficiency and that activities of certain mutant LCAT proteins derived from FLD and FED patients were enhanced (Non Patent Literature 19).

Pyrazolopyridine-skeleton compounds described in Patent Literatures 2 to 4 are known as low molecular compounds having an activation effect on normal human LCAT proteins. However, it is unknown so far whether the pyrazolopyridine-skeleton compounds have a good LCAT activation effect on mutant LCAT proteins derived from patients.

### Citation List

### Patent Literature

Patent Literature 1: WO2008/002591
Patent Literature 2: WO2012/028243
Patent Literature 3: WO2013/187462
Patent Literature 4: WO2015/087994

### Non Patent Literature

Non Patent Literature 1: Kuivenhoven, J.A., J. Lipid Res., 1997, vol. 38, 191-205
Non Patent Literature 2: Miettinen, H.E., Arterioscler. Thromb. Vasc. Biol., 1998, vol. 18, 591-598
Non Patent Literature 3: Nanjee, M.N., Atherosclerosis, 2003, vol. 170, 105-113
Non Patent Literature 4: Frasca, G.M., Nephrol. Dial. Transplamt., 2004, vol. 19, 622-624
Non Patent Literature 5: Hirashio, S., J. Arterioscler. Thromb., 2010, vol. 17, 1297-1301
Non Patent Literature 6: Wang, X.L., J. Arterioscler. Thromb., 2011, vol. 18, 713-719
Non Patent Literature 7: Roshan, B., J. Clin. Lipidol., 2011, vol. 5, 493-499
Non Patent Literature 8: Conca, P., J. Clin. Lipidol., 2012, vol. 6, 244-250
Non Patent Literature 9: Naito, S., Atherosclerosis, 2013, vol. 228, 193-197
Non Patent Literature 10: Castro-Ferreira, I., JIMD., Rep., 2018, vol. 40, 55-62
Non Patent Literature 11: Oliaei, F., J. Cell Biochem., 2018, vol. 2
Non Patent Literature 12: Blanco-Vaca, F., Atherosclerosis, 1997, vol. 131, 85-95
Non Patent Literature 13: Miida, T., Clin. Chim. Acta., 2004, vol. 343, 201-208
Non Patent Literature 14: Kuroda, M., Nippon Rinsho, 2013, vol. 71, extra edition 3, 275-279
Non Patent Literature 15: Shamburek, R., J. Clin. Lipidol., 2016, vol. 10, 356-367
Non Patent Literature 16: Chen, Z., J. Cardiovasc. Transl. Res., 2011, vol. 4, 801-810
Non Patent Literature 17: Kuroda, M., J. Diabet. Invest., 2011, vol. 2, 333-340
Non Patent Literature 18: Iwata, A., Atherosclerosis, 2011, vol. 218, 300-307
Non Patent Literature 19: Freeman, L.A., J. Pharmacol. Exp. Ther., 2017, vol. 362, 306-318

### Summary of Invention

### Technical Problem

At present, there is a report on a low molecular compound showing a LCAT activation effect on patient's blood with a LCAT mutation but the compound is not satisfactory in the aspects of safety and effectiveness. There is a serious demand for a safe and effective LCAT activator that can be continuously administered for an extended period of time to a patient with LCAT deficiency, which is a genetic disease, for the purpose of preventing transition to dialysis due to the progress of renal function decrease or transition to corneal transplantation due to the progress of corneal opacity.

### Solution to Problem

The present inventors found that pyrazolopyridine derivatives having a specific structure or a pharmacologically acceptable salt thereof has a good LCAT activation effect on certain mutant LCAT proteins derived from patients.

The present invention provides a therapeutic drug for LCAT deficiency containing a pyrazolopyridine derivative or a pharmacologically acceptable salt thereof having a good LCAT activation effect (preferably a reversible LCAT activation effect) directly on an endogenous mutant LCAT protein of a patient himself/herself.

### More specifically, the present invention provides

(1) a therapeutic drug for LCAT deficiency comprising a compound represented by Formula (I)

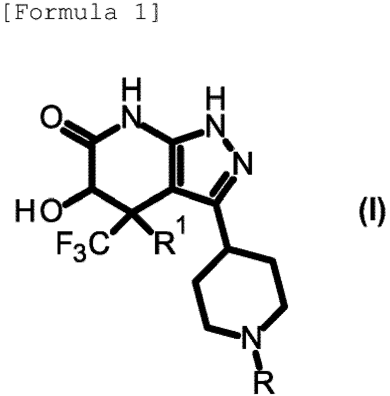

   wherein R¹ is a hydrogen atom or a hydroxyl group, and R is a 2-(trifluoromethyl)pyrimidin-5-yl group or a 5-(trifluoromethyl)pyrazin-2-yl group, or a pharmacologically acceptable salt thereof,
(2) the therapeutic drug for LCAT deficiency according to (1), wherein R¹ is a hydrogen atom, and R is a 2-(trifluoromethyl)pyrimidin-5-yl group,
(3) the therapeutic drug for LCAT deficiency according to (1), wherein R¹ is a hydroxyl group, and R is a 5-(trifluoromethyl)pyrazin-2-yl group,
(4) the therapeutic drug for LCAT deficiency according to (1) to (3), wherein the LCAT deficiency is familial LCAT deficiency (FLD),
(5) the therapeutic drug for LCAT deficiency according to (1) to (3), wherein the LCAT deficiency is FLD caused by an amino acid mutation G30S, L32P, G33R, A93T, R135W, R135Q, R140H, R147W, Y156N, G183S, L209P, N228K, R244G, M252K, T321M, G344S, T347M, R399C, G230R, F382V, T208S, S181N, R140C, G179R, M293R, P406L, C74T, R268L, or D101N,
(6) the therapeutic drug for LCAT deficiency according to (1) to (3), wherein the LCAT deficiency is FLD caused by an amino acid mutation R147W, T274I, L372R, P278S, V309M, Y156N, or N228K,
(7) the therapeutic drug for LCAT deficiency according to (1) to (3), wherein the LCAT deficiency is fish eye disease (FED),
(8) the therapeutic drug for LCAT deficiency according to (1) to (3), wherein the LCAT deficiency is FED caused by an amino acid mutation N131D, N391S, P10L, P10Q, T123I, R99C, or T13M,
(9) the therapeutic drug for LCAT deficiency according to (1) to (3), wherein the LCAT deficiency is FED caused by an amino acid mutation N131D, P10Q, or T123I, and
(10) a method for treating LCAT deficiency comprising a step of administering an effective dose of the compound or a pharmacologically acceptable salt thereof according to any one of (1) to (9) to a human.

Hereinafter, the compound (I) of the present invention is described.

The compound (I) of the present invention includes both a compound represented by Formula (I), and a tautomer thereof, which is a compound represented by Formula (Ix).

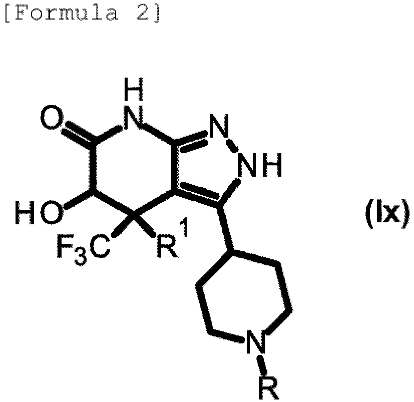

In the present application, the compound (I) including any tautomers is for convenience represented by the structural formula of Formula (I) and the chemical name corresponding thereto unless otherwise specified. Further, any isomers of other tautomers (amide-imide acid) of the compound (I) of the present invention are included in the compound (I) of the present application, and in the present application, the compound (I) including any tautomers is for convenience represented by the structural formula of Formula (I) and the chemical name corresponding thereto.

The compound (I) of the present invention has a basic group and hence can form an acid addition salt with a pharmacologically acceptable acid. In the present invention, examples of the "a pharmacologically acceptable salt thereof" include hydrohalides such as hydrofluoride, hydrochloride, hydrobromide, and hydroiodide; inorganic acid salts such as nitrate, perchlorate, sulphate, and phosphate; lower alkanesulfonates such as methanesulfonate, trifluoromethanesulfonate, and ethanesulfonate; arylsulfonates such as benzenesulfonate and p-toluenesulfonate; organic acid salts such as acetic acid, malic acid, fumarate, succinate, citrate, tartrate, oxalate, and maleate; and amino acid salts such as ornithate, glutamate, and aspartate.

The compound (I) of the present invention or a pharmacologically acceptable salt thereof absorbs moisture when left in the atmosphere and may become a hydrate, and such a hydrate is also encompassed in the present invention.

The compound (I) of the present invention or a pharmacologically acceptable salt thereof may become a solvate when left in a solvent, and such a solvate is also encompassed in the present invention.

In the compound (I) of the present invention, optical isomers are present around the asymmetric center in the molecule. In the compound of the present invention, these isomers and mixtures of these isomers are all represented by a single formula, more specifically, Formula (I), unless otherwise specified. Thus, the present invention should include all these isomers and mixtures of these isomers.

The compound (I) of the present invention can also include a non-natural abundance ratio of an atomic isotope at one or more atoms making up the compound. Examples of atomic isotopes include deuterium (²H), tritium (³H), iodine-125 (¹²⁵I), and carbon-14 (¹⁴C) . Further, the above compound can be radioactively labelled with a radioisotope such as tritium (³H), iodine-125 (¹²⁵I) , or carbon-14 (¹⁴C) . The radioactively labelled compound is useful as a therapeutic or preventive agent, a research reagent such as an assay reagent, and a diagnostic agent such as an in-vivo image diagnostic agent. All isotopic variants of the compound of the present invention should be encompassed within the scope of the present invention regardless of being radioactive or not.

In the present invention, LCAT deficiency includes deficiency of a gene encoding LCAT, decreased expression of LCAT, and inactivation of LCAT. The deficiency of a gene encoding LCAT includes homozygous deficiency and heterozygous deficiency. The decreased expression of LCAT includes both decreased expression at transcriptional level and decreased expression at translational level. The inactivation of LCAT typically includes an inactive mutation of a gene encoding LCAT. Such LCAT deficiencies are roughly categorized into familial LCAT deficiency (FLD) and fish eye disease (FED). Examples of the amino acid mutation of FLD include G30S, L32P, G33R, A93T, R135W, R135Q, R140H, R147W, Y156N, G183S, L209P, N228K, R244G, M252K, T321M, G344S, T347M, R399C, G230R, F382V, T208S, S181N, R140C, G179R, M293R, P406L, C74T, R268L, and D101N, and examples of the amino acid mutation of FED include N131D, N391S, P10L, P10Q, T123I, R99C, and T13M.

### Advantageous Effects of Invention

The compound represented by Formula (I) of the present invention or a pharmacologically acceptable salt thereof has a good LCAT activation effect on LCAT mutant proteins derived from patients and is useful as a therapeutic agent for LCAT deficiency.

### Description of Embodiments

The compound (I) of the present invention can be produced by the method described in WO2015/087994.

A pharmacologically acceptable salt of the compound (I) of the present invention can be produced by carrying out a routine salt-forming reaction. Isolation and purification are carried out by applying a routine chemical operation such as extraction, concentration, distillation, crystallization, filtration, recrystallization, or various chromatographies.

Different isomers of the compound (I) of the present invention can be separated by utilizing a difference in physico-chemical properties among isomers. For example, a racemic mixture can be led to an optically pure isomer by fractional crystallization leading to a diastereomeric salt with an optically active base or acid or by chromatography using a chiral column. Additionally, a diastereomer mixture can be separated by fractional crystallization or by various chromatographies. Additionally, an optically active compound can also be produced by using a suitable optically active raw material.

Examples of dosage forms of the compound having Formula (I) of the present invention or a pharmacologically acceptable salt thereof include oral administration using a tablet, a granule, a powder, a capsule, a syrup or the like, and parenteral administration using an injection, a suppository or the like, and the compound or a salt thereof can be administered systemically or locally.

Examples of the form of oral pharmaceutical drug of the compound having Formula (I) of the present invention or a pharmacologically acceptable salt thereof include a tablet, a pill, a granule, a powder, a capsule, a liquid, a suspension, an emulsion, a syrup, and an elixir. Examples of the form of parenteral pharmaceutical drug include an injection, an ointment, a gel, a cream, a patch, an aerosol, an inhaler, a spray, an eye drop, and a suppository. Pharmaceutical drugs of these forms can be prepared in accordance with routine methods using an additive suitably selected as needed from pharmaceutically acceptable additives such as an excipient, a binder, a diluent, a stabilizer, a preservative, a coloring agent, a solubilizer, a suspending agent, a buffer, or a wetting agent.

The dosage of the compound having Formula (I) of the present invention or a pharmacologically acceptable salt thereof when administered varies depending on symptoms, body weight, age, administration method, and the like of a subject to be administered (a warm-blooded animal such as a human). For example, in the case of oral administration, it is desirable to administer, according to symptoms, 0.01 mg/kg of body weight (preferably 0.03 mg/kg of body weight) as the lower limit and 300 mg/kg of body weight (preferably 100 mg/kg of body weight) as the upper limit per administration from 1 to several times a day. Further, in the case of intravenous administration, it is desirable to administer, according to symptoms, 0.01 mg/kg of body weight (preferably 0.03 mg/kg of body weight) as the lower limit and 300 mg/kg of body weight (preferably 100 mg/kg of body weight) as the upper limit per administration from 1 to several times a day.

Hereinafter, the present invention is described in further detail with reference to examples, test examples, and preparation examples, but the scope of the present invention is not limited thereto.

### Examples

### (Test compound) (+)-Cis-5-hydroxy-4-(trifluoromethyl)-3-{1-[2-(trifluoromethyl)pyrimidin-5-yl]piperidin-4-yl}-1,4,5,7-tetrahydro-6H-pyrazolo[3,4-b]pyridin-6-one

The test compound was produced in accordance with the method of Example 21 described in WO2015/087994.

### (Text Example 1) Measurement of LCAT activity using LCAT mutant proteins

Mutant LCAT proteins, reported on gene mutations among patients with familial LCAT deficiency (FLD) and patients with fish eye disease (FED), were expressed in HEK293 cells, and the mutant LCAT proteins were purified from the culture supernatant and used as enzyme sources. HDL was reconstituted with phosphatidylcholine, [14C]-labelled cholesterol, and apolipoprotein A-I and used as a reactive substrate. The test compound was prepared by being dissolved in dimethyl sulfoxide. To a mixture of 7 µL of the mutant LCAT protein solution (concentration 0.034 µg/µL), 1 µL each of PBS containing 10% bovine serum albumin or mercaptoethanol, and 40 µL of the above-mentioned reconstituted HDL was further added 1 µL of the test compound (final concentration 1 or 10 µM) to prepare 50 µL of a mixture in total. The mixture was incubated at 37°C for 1 hour and subsequently a mixed solution of hexane and isopropanol (mixing ratio = 3:2) was added to stop the reaction. Water was added and stirred, and subsequently the hexane layer was collected and concentrated to dryness. A chloroform solution (concentration 10 mg/mL) was added thereto, spotted on a silica gel thin-layer plate and developed with a mixed solution of hexane, diethyl ether and ethyl acetate (mixing ratio = 85:15:2). Radioactivity of a portion corresponding to the cholesterol ester separated on each lane by the development on the silica gel thin-layer plate was measured using an imaging analyzer BAS-2500 (manufactured by FUJIFILM Corporation). Radioactivities with and without the test compound were compared to calculate the ratio of LCAT activity with the addition of the test compound to that without the addition of the test compound for each of the mutant proteins. Additionally, the presence and absence of significant differences in both cases were statistically evaluated using t-test or Dunnett multiple comparison test. The results are shown in Table 1.

**[Table 1]**

| Disease | Mutant protein | Compound concentration (µM) | Measured value of LCAT activity Mean ± standard error (n = 6) | LCAT activity ratio of case of compound addition to no-addition control | p Value |
|---|---|---|---|---|---|
| FLD | Y156N | 0 | 244.03 ± 23.12 | 1.00 | - |
| | | 1 | 447.36 ± 47.06 | 1.83 | 0.0028 |
| | | 10 | 443.20 ± 36.83 | 1.82 | 0.0033 |
| | N228K | 0 | 226.57 ± 18.89 | 1.00 | - |
| | | 1 | 634.17 ± 66.62 | 2.80 | <0.0001 |
| | | 10 | 625.22 ± 41.03 | 2.76 | <0.0001 |
| FED | P10Q | 0 | 760.17 ± 53.37 | 1.00 | - |
| | | 1 | 2908.25 ± 429.42 | 3.83 | 0.0039 |
| | T1231 | 0 | 869.83 ± 56.38 | 1.00 | - |
| | | 1 | 3263.11 ± 521.27 | 3.75 | 0.0057 |
| | N131D | 0 | 409.84 ± 57.36 | 1.00 | - |
| | | 1 | 1308.38 ± 259.22 | 3.19 | 0.0169 |

As evident above, statistically significant enhancement effects as to LCAT activity on the mutant LCAT proteins were demonstrated by treatment with the test compound, which is thereby useful as a pharmaceutical drug for treating or preventing LCAT deficiency disease.

### (Test Example 2) Measurement of LCAT activity using LCAT mutant proteins

In the present invention, LCAT deficiency (LCAT function disorder) is roughly categorized into familial LCAT deficiency (FLD) and fish eye disease (FED). Examples of the amino acid mutation of FLD include, in addition to Y156N, and N228K, G30S, L32P, G33R, A93T, R135W, R135Q, R140H, R147W, G183S, L209P, R244G, M252K, T321M, G344S, T347M, R399C, G230R, F382V, T208S, S181N, R140C, G179R, M293R, P406L, C74T, R268L, and D101N, and examples of the amino acid mutation of FED include, in addition to P10Q, T123I, and N131D, N391S, P10L, R99C, and T13M.

Tests can be carried out in the same manner as in Test Example 1 using the above mutant LCAT proteins in place of the mutant LCAT proteins used in Test Example 1, thereby confirming LCAT activity enhancement using other LCAT mutant proteins.

### (Preparation Example 1) Hard capsule

A unit capsule is produced by filling each of standard bisect hard gelatin capsules with 100 mg of powder Example compound, 150 mg of lactose, 50 mg of cellulose, and 6 mg of magnesium stearate, washed and then dried.

### (Preparation Example 2) Soft capsule

A mixture of Example compound placed in a digestible oily matter such as soybean oil, cottonseed oil, or olive oil is prepared and injected into a gelatin using a positive displacement pump to obtain a soft capsule containing 100 mg of the active ingredient, and the capsule is washed and then dried.

### (Preparation Example 3) Tablet

A tablet is produced in accordance with a routine method using 100 mg of Example compound, 0.2 mg of colloidal silicon dioxide, 5 mg of magnesium stearate, 275 mg of microcrystalline cellulose, 11 mg of starch, and 98.8 mg of lactose.

A coating is applied as desired.

### (Preparation Example 4) Suspension

A 5-mL suspension is produced in such a way as to contain 100 mg of micropowdered Example compound, 100 mg of carboxymethyl cellulose sodium, 5 mg of sodium benzoate, 1.0 g of a sorbitol solution (Japanese Pharmacopoeia), and 0.025 mL of vanillin.

### (Preparation Example 5) Injection

In 10 wt% of propylene glycol is stirred 1.5 wt% of Example compound and subsequently the resulting mixture is adjusted to a fixed volume with water for injection, followed by sterilization for use as an injection.

### Industrial Applicability

As shown in Test Example 1, the compound represented by Formula (I) of the present invention or a pharmacologically acceptable salt thereof can recover LCAT activity diminished among patients with LCAT deficiency for itself by demonstrating a good activity enhancing effect on mutant LCAT proteins sensitive thereto.

Gene mutation in LCAT deficiency is diversified. Even when the compound represented by Formula (I) of the present invention or a salt thereof encounters a LCAT mutation failing to express sufficient sensitivity, the compound represented by Formula (I) of the present invention or a pharmacologically acceptable salt thereof is used in combination with enzyme replacement therapy by a recombinant LCAT protein preparation, gene therapy using an adeno-associated virus vector currently under development or ex vivo gene and cell therapy using adipocytes, thereby enhancing the activity of the LCAT protein remaining in the blood despite exogenous protein clearance in vivo, the appearance of antibodies to the exogenous protein, or decreased exogenous LCAT activity caused by decreased expression of the LCAT transgene, whereby stable retention of LCAT activity, which can reduce dose and frequency, etc. in enzyme replacement, and the consequent high therapeutic effect can be achieved.

Thus, the compound of the present invention or a salt thereof is useful as an effective ingredient of a therapeutic or preventive agent for corneal opacity or kidney disease associated with LCAT deficiency.

## Claims

1. A therapeutic drug for LCAT deficiency comprising a compound represented by Formula (I) 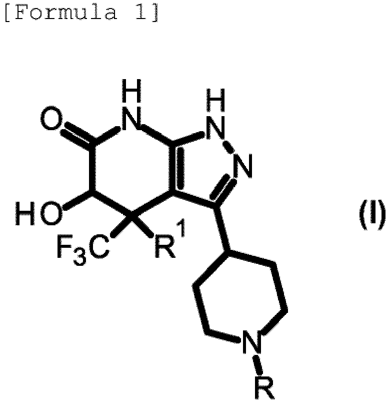 wherein R¹ is a hydrogen atom or a hydroxyl group, and R is a 2-(trifluoromethyl)pyrimidin-5-yl group or a 5-(trifluoromethyl)pyrazin-2-yl group, or a pharmacologically acceptable salt thereof.

2. The therapeutic drug for LCAT deficiency according to claim 1, wherein R¹ is a hydrogen atom, and R is a 2-(trifluoromethyl)pyrimidin-5-yl group.

3. The therapeutic drug for LCAT deficiency according to claim 1, wherein R¹ is a hydroxyl group, and R is a 5-(trifluoromethyl)pyrazin-2-yl group.

4. The therapeutic drug for LCAT deficiency according to any one of claims 1 to 3, wherein the LCAT deficiency is familial LCAT deficiency (FLD).

5. The therapeutic drug for LCAT deficiency according to any one of claims 1 to 3, wherein the LCAT deficiency is FLD caused by an amino acid mutation G30S, L32P, G33R, A93T, R135W, R135Q, R140H, R147W, Y156N, G183S, L209P, N228K, R244G, M252K, T321M, G344S, T347M, R399C, G230R, F382V, T208S, S181N, R140C, G179R, M293R, P406L, C74T, R268L, or D101N.

6. The therapeutic drug for LCAT deficiency according to any one of claims 1 to 3, wherein the LCAT deficiency is FLD caused by an amino acid mutation Y156N or N228K.

7. The therapeutic drug for LCAT deficiency according to any one of claims 1 to 3, wherein the LCAT deficiency is fish eye disease (FED).

8. The therapeutic drug for LCAT deficiency according to any one of claims 1 to 3, wherein the LCAT deficiency is FED caused by an amino acid mutation N131D, N391S, P10L, P10Q, T123I, R99C, or T13M.

9. The therapeutic drug for LCAT deficiency according to any one of claims 1 to 3, wherein the LCAT deficiency is FED caused by an amino acid mutation N131D, P10Q, or T123I.

10. A method for treating LCAT deficiency comprising a step of administering an effective dose of the compound or a pharmacologically acceptable salt thereof according to any one of claims 1 to 9 to a human.
